# EUROPEAN PATENT APPLICATION

(11) **EP 4 357 331 A1**
(43) Date of publication of application: **24.04.2024**
(21) Application number: 22825352.2
(22) Date of filing: 16.06.2022
(51) Int. Cl.: C07C 51/47, C07C 59/01, C12P 7/52, B01D 15/36

(54) **METHOD FOR OBTAINING DESIRED COMPOUND FROM FERMENTATION BROTH**

(30) Priority: 16.06.2021 KR 20210078346
(71) Applicant: GS Caltex Corporation, Seoul 06141 (KR)
(72) Inventor: UEOM, Moon-Ho, Daejeon 34122 (KR); JANG, Jun-Ho, Daejeon 34122 (KR); JEON, Sang-Jun, Daejeon 34122 (KR); LEE, Kyoung-Jun, Daejeon 34122 (KR)
(74) Representative: Zacco Sweden AB
(86) International application number: PCT/KR2022/008552
(87) International publication number: WO 2022/265429

(57) **Abstract**

The present invention relates to a method for obtaining a compound, after removing at least a portion of insoluble substances or macromolecules from a fermentation broth, by performing one or more of the following steps (a) to (c), and recovering a desired compound: (a) increasing the concentration of a desired compound in the fermentation broth; (b) reducing the concentration of a residual carbon source or alcohol in the fermentation broth; and (c) reducing the concentration of ionic components in the fermentation broth.

## Description

### FIELD

The present disclosure relates to a method for obtaining a target compound from a fermentation broth.

### DESCRIPTION OF RELATED ART

3-hydroxypropionic acid (3-HP) may be produced through a chemical synthesis process and a microorganism fermentation process, and recently, a method of producing 3-HP using bacteria such as Escherichia coli and Klebsiella has been studied. For example, Korean Patent Application Publication No. 10-2020-0051375 discloses a technique for producing 3-HP using a microorganism transformed with a specific gene.

The present disclosure relates to a method for obtaining 3-HP by separating 3-HP from a fermentation broth containing 3-HP produced by microbial culture.

### DISCLOSURE

### TECHNICAL PURPOSE

It is a purpose of the present disclosure to provide a method for obtaining a target compound from a fermentation broth.

### TECHNICAL SOLUTION

In order to achieve the purpose, the present disclosure provides: a method for obtaining a target compound from a fermentation broth, the method comprising: removing at least a portion of an insoluble substance or a macromolecule from the fermentation broth; performing at least one of following steps (a) to (c):
(a) increasing a concentration of the target compound in the fermentation broth;
(b) reducing a concentration of a residual carbon source or alcohol in the fermentation broth; and
(c) reducing a concentration of an ionic component in the fermentation broth; and
collecting the target compound from the fermentation broth.

### TECHNICAL EFFECT

According to the method of the present disclosure, the target compound may be separated from the fermentation broth at a high yield.

### BRIEF DESCRIPTION OF THE DRAWINGS

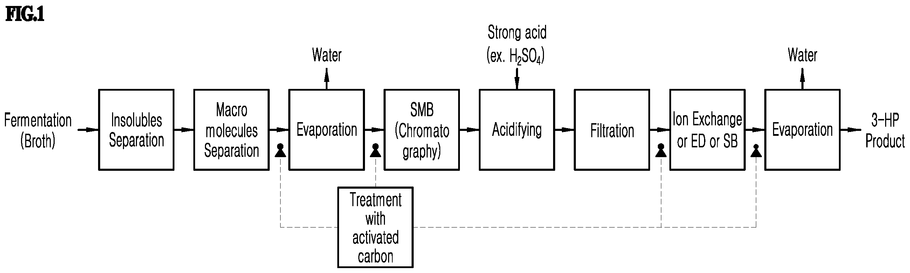

FIG. 1 is a schematic view showing a compound harvesting method of the present disclosure.
FIG. 2 is a test result obtained by separating an insoluble substance from a fermentation broth through a micro-filter.
FIG. 3 shows a result of identifying peaks of 3-HP, 1,3-propanediol, and glycerol in a fermentation broth via single column chromatography.
FIG. 4 illustrates a process of precipitating and removing calcium ions in a fermentation broth.
FIG. 5 shows change in an ion concentration in a fermentation broth according to ion exchange.
FIG. 6 illustrates a process of removing ions in a fermentation broth via ion exchange.
FIG. 7 illustrates a process of removing ions in a fermentation broth via electrodialysis.

### DETAILED DESCRIPTIONS

The present disclosure relates to a method for obtaining a target compound from a fermentation broth, the method comprising: removing at least a portion of an insoluble substance or a macromolecule from the fermentation broth; performing at least one of following steps (a) to (c):
(a) increasing a concentration of the target compound in the fermentation broth;
(b) reducing a concentration of a residual carbon source or alcohol in the fermentation broth; and
(c) reducing a concentration of an ionic component in the fermentation broth; and
collecting the target compound from the fermentation broth.

In this regard, when one or more steps of (a) to (c) are performed, any step may be repeated twice or more.

Step (a) may be performed through at least one selected from a group consisting of evaporation, concentration, and distillation.

Step (c) may be carried out by a following (c)' or (c)":
(c)': a process of acidifying the fermentation broth to precipitate the ionic component and then removing the precipitated ionic component; and
(c)": A process of treating the fermentation broth with at least one selected from the group consisting of electrodialysis, ion exchange, and chromatography.

The insoluble substance may include cells, and the macromolecule may be a protein, a polysaccharide, or a lipid. In this regard, at least a portion of the insoluble substance may be removed from the fermentation broth, at least a portion of the macromolecule may be removed therefrom, or at least a portion of the insoluble substance and at least a portion of the macromolecule may be removed therefrom. In this regard, at least a portion of the insoluble substance may be removed from the fermentation broth, and then, at least a portion of the macromolecule may be removed therefrom. Alternatively, at least a portion of the macromolecule may be removed from the fermentation broth, and then, at least a portion of the insoluble substance may be removed therefrom. The target compound may be an organic acid produced by microbial fermentation, and preferably 3-hydroxypropionic acid (3-HP). The carbon source of the (b) may be glycerol or glucose. The alcohol of the (b) may be an alcohol produced by microbial fermentation, for example, 1,3-propanediol or the like.

In a preferred embodiment of the present disclosure, the method for preparing the compound of the present disclosure may remove at least a portion of an insoluble substance from the fermentation broth, and may remove at least a portion of the macromolecule therefrom, and then, (a) may first increase the concentration of the target compound in the fermentation broth, and (b) may reduce the concentration of the remaining carbon source or alcohol in the fermentation broth, and c)' may acidify the fermentation broth to precipitate ionic components and then remove the pre precipitated ionic components, and (c)" may treat the fermentation broth with at least one selected from the group consisting of electrodialysis, ion exchange, and chromatography, and then (a) may second increase the concentration of the target compound in the fermentation broth, and may collect the target compound from the fermentation broth.

The fermentation broth may be a fermentation broth prepared in a following process 1: a fermentation broth preparation step.

The step of removing at least a portion of the insoluble substance or the macromolecule from the fermentation broth may be a following process 2: separation step of insoluble material or a following process 3: macromolecule removal and decolorizing step.

The step (a) of first increasing the concentration of the target compound in the fermentation broth may be a step of increasing the concentration of 3-HP in the fermentation broth.

The step (b) of reducing the concentration of the residual carbon source or alcohol in the fermentation broth may be a step of reducing the concentration of glycerol or alcohol in the fermentation broth.
(c)': the step of acidifying the fermentation broth to precipitate the ionic component and then removing the precipitated ionic component may be a step (precipitation and filtration) of reducing the content of the ionic component in the fermentation broth.
(c)": the step of treating the fermentation broth with at least one selected from the group consisting of electrodialysis, ion exchange, and chromatography may be a step (electrodialysis and ion exchange) of reducing an ionic component in the fermentation broth.

The step (a) of second increasing the concentration of the target compound in the fermentation broth may be a step of increasing the concentration of 3-HP in the fermentation broth.

The collecting of the target compound may be a following process 9: collection step.

### Process 1: Preparation of Fermentation Broth

The method for preparing the compound of the present disclosure may include a fermentation broth preparation step. The fermentation broth may be a fermentation broth fermented using microorganisms, that is, a culture medium. The culture medium may further include other components other than 3-HP and the salt thereof. For example, the culture medium may include a calcium salt of 3-HP. The other components may be carbon sources (e.g. glycerol, etc.), organic acids (such as acetic acid, etc.), salts (e.g. sulfates, phosphates, etc.), alcohols (such as diols), sugars, calcium, etc. used in microbial culture.

The concentration of 3-HP or salt thereof in the fermentation broth may be, for example, in a range from about 10,000 to 200,000 ppm, such as from about 12,000 to 190,000 ppm, such as from about 15,000 to 180,000 ppm, such as from about 20,000 to 150,000 ppm, such as from about 25,000 to 130,000 ppm, such as from about 30,000 to 110,000 ppm. In some embodiments, the concentration of 3-HP or salt thereof in the fermentation broth may be, for example, in a range of at least about 10,000 ppm, such as at least about 12,000 ppm, such as at least about 15,000 ppm, such as at least about 20,000 ppm, such as at least about 25,000 ppm, such as at least about 30,000 ppm. In embodiments, the concentration of 3-HP or salt thereof in the fermentation broth may be, for example, in a range of 200,000 ppm or smaller, such as about 190,000 ppm or smaller, such as about 180,000 ppm or smaller, such as about 150,000 ppm or smaller, such as about 130,000 ppm or smaller, such as about 110,000 ppm or smaller.

The method may further include adjusting a pH of the fermentation broth. The pH may be adjusted by treating the fermentation broth with an acid or alkali. The type of acid or alkali is not particularly limited, and a common acid or alkali used when the pH of the fermentation broth is adjusted may be used. For example, the target pH may be appropriately adjusted according to 3-HP, and types of compounds to be separated therefrom, and a method of a subsequent separation process.

### Process 2: Separation of Insoluble Substance

The method for obtaining the compound of the present disclosure may include separating at least a portion of the insoluble substance from the fermentation broth. In this regard, the fermentation broth may be a fermentation broth prepared in Process 1 or a fermentation broth obtained by being subjected to Processes 1 and 3, that is, a treated fermentation broth. The insoluble substance may be a microorganism, i.e. a cell, a solid, or the like.

The separation of the insoluble substance may be performed using a centrifuge, a filtration process, or the like, but is not particularly limited. Thus, the microorganism, i.e. cells, may be separated from the fermentation broth.

For example, solids in the microorganism and the fermentation broth may be removed therefrom through microfiltration. The microfiltration may be performed by passing the fermentation broth through a polymer or ceramic membrane having a pore size of 0.04 to 5 µm. In this case, the microfiltration operation may be implemented in two or more steps due to the tendency of decrease in a flux (the throughput of the membrane per unit time and the unit area). In one embodiment, water may be further added to the fermentation broth to increase the collection rate at a point in time when the flux is significantly lowered.

### Process 3: Macromolecules Removal and Decolorizing Step

The method for obtaining the compound of the present disclosure may include separating at least a portion of the macromolecule from the fermentation broth. In this regard, the step of separating at least a portion of the macromolecule from the fermentation broth may include a step of removing the macromolecules from the fermentation broth and decolorizing the same. In this regard, the fermentation broth may be a fermentation broth prepared in Process 1 or a fermentation broth obtained by being subjected to Processes 1 and 2, that is, a treated fermentation broth. The macromolecules may be proteins, polysaccharides, lipids, and the like.

In one embodiment, the macromolecule removing and decolorizing step may be performed by mixing activated carbon and a filtration aid with the fermentation broth and filtering the same. Specifically, in one embodiment, the activated carbon and the filtering aid (diatomaceous earth, white clay, etc.) may be mixed with the fermentation broth from which the microorganisms have been removed, and then the mixture may be subjected to filtering (for example, a filter press) to obtain a macromolecule removal and decolorization effect. The filtering method (for example, a filter press, a drum filter, a candle filter, a leaf filter, etc.) of powder particles that may be commonly used may be used for the filtration. However, the filtering method is not particularly limited thereto.

Removing macromolecules such as some proteins, polysaccharides, etc. through addition of activated carbon may allow production of adhesive materials that may occur in the subsequent process of increasing the 3-HP concentration to be suppressed. the activated carbon may include granular activated carbon as well as powdered activated carbon. For example, 0.1 to 2 wt % of powdered activated carbon was added to the fermentation broth with respect to the weight of the fermentation broth, and the mixture was stirred for 1 to 5 hours under the condition of 30 to 80°C, and was filtered to remove the color of the fermentation broth. After mixing 1.5% of powdered activated carbon with the fermentation broth, the mixture was stirred at 40°C for 1 hour and was subjected to filtering. In this case, a APHA (American Public Health Association) color value of the fermentation broth was 2.7. In addition, when the granular activated carbon is added at a mass fraction of 5 to 15% with respect to the weight of the fermentation broth, decolorizing performance similar to that in use of the powdered activated carbon may be expected, and in this case, there is a need to be equipped with a recycling system of the activated carbon using steam or the like in order to secure economic feasibility.

In another embodiment, the macromolecule removal and decolorization step may be performed through membrane separation of the treated fermentation broth. In this case, the membrane separation method may be ultrafiltration or nanofiltration, and in this case, macromolecular removal and decolorizing effects may be obtained without an additional decolorizing process.

In this case, the ultrafiltration using the ultrafiltration membrane having a molecular weight ratio of cut-off (MWCO) of 5000 to 100,000 Da may be applied, and then, the nanofiltration using a nano-filtration membrane having a molecular weight ratio of cut-off (MWCO) of 150 to 300 Da may be applied, thereby removing only macromolecules such as proteins, polysaccharides, lipids, and the like while minimizing the loss of 3-HP salts.

### Process 4: Increasing Concentration of 3-HP in Fermentation Broth

The method for obtaining the compound of the present disclosure may include a step of increasing the concentration of 3-HP in the fermentation broth. In this case, the fermentation broth of Process 4 may be a treated fermentation broth that has been subjected to Process 2 and/or Process 3. Alternatively, the fermentation broth of Process 4 may be a treated fermentation broth that has been subjected to Process 2 and/or Process 3 and then, at least one of Processes 5 to 8. In this case, at least a portion of the liquid in the fermentation broth may be evaporated. Alternatively, the concentration of 3-HP may be increased by a method such as concentrating. In this regard, the concentration of 3-HP may be increased to the concentration of 100 to 500 g of 3-HP equivalent per 1 L of the fermentation broth. The increase in the concentration may involve pressure regulation, and may be achieved under appropriate pressure, such as from about 20 to 300 mbar, such as from about 25 to 280 mbar, such as from about 30 to 250 mbar, such as from about 50 to 200 mbar, such as from about 70 to 190 mbar, or such as from about 80 to 180 mbar based on an absolute pressure. In one embodiment the concentration increasing step may be performed, for example, at a pressure in a range of about 20 mbar or lower, such as about 25 mbar or lower, such as about 30 mbar or lower, such as about 50 mbar or lower, such as about 70 mbar or lower, or such as about 80 mbar or lower. In one embodiment, the concentration increasing step may be performed, for example, at a pressure in a range of about 300 mbar or lower, such as about 280 mbar or lower, such as about 250 mbar or lower, such as about 200 mbar or lower, such as about 190 mbar or lower, such as about 180 mbar or lower.

For example, while under reduced pressure of 50 mbar, a bath temperature of a reactor was maintained at 50°C, the concentration was carried out until the concentration of 3-HP was 500 g/L or higher. In a concentration condition of 400 g mol/L or higher, it was identified that a crystal of a 3-HP calcium salt was produced due to a decrease in the temperature after concentration termination. It was identified that the crystal was produced in a concentration process as the concentration increased to a value above 400 g mol/L or higher.

### Process 5: Reducing Concentration of Glycerol or Alcohol in Fermentation Broth

The method for obtaining the compound of the present disclosure may include a step of reducing the concentration of glycerol or alcohol in a fermentation broth. In this case, the fermentation broth of Process 5 may be a treated fermentation broth that has been subjected to Process 2 and/or Process 3. Alternatively, the fermentation broth of Process 5 may be a treated fermentation broth that has been subjected to Process 2 and/or Process 3 and then, at least one of Process 4 or Processes 6 to 8. The glycerol may be a carbon source used when culturing microorganisms, and the alcohol may be a culture product of microorganisms, such as 1,3-propanediol (1,3-PDO). When glucose is used as the carbon source, the concentration of glucose in the fermentation broth may be reduced by the process 5. In this regard, the concentration of glycerol or alcohol in the fermentation broth may be reduced by applying chromatography using an ion-exchange resin (for example, SMB-MS (simulated moving bed tar) chromatography). The concentration of glycerol or alcohol in the fermentation broth is an important factor affecting the quality of a product when performing polymer polymerization using 3-HP obtained by the method of the present disclosure. Using chromatography or SMB chromatography, the mobile phase may be injected together with the feed to obtain at least one fraction (fraction A) rich in 3-HP and at least one fraction (fraction B) rich in a compound other than 3-HP.

### Process 6: Reducing Ionic Components in Fermentation Broth (Precipitation and Filtration

A method for obtaining the compound of the present disclosure may include a step of reducing an ionic component in the fermentation broth. In this case, the fermentation broth of Process 6 may be a treated fermentation broth that has been subjected to Process 2 and/or Process 3. Alternatively, the fermentation broth of Process 6 may be a treated fermentation broth that has been subjected to Process 2 and/or Process 3 and then, at least one of Processes 4, 5, 7, and 8. In one embodiment, when the process 5 has been performed, the largest impurity in the fermentation broth in which the content of the glycerol and alcohol has been reduced is a cation of the pH regulator introduced for controlling pH in the step of preparing the fermentation broth. Depending on the separation and purification process, different pH regulators may be used, and thus the different types of cations may be present. Generally, the cation may be cations of calcium, magnesium, or the like. However, the present disclosure is not limited thereto. These cations present in the form of various 3-HP salts via a weak chemical bond of the cations with 3-HP may be efficiently removed in a form of precipitates via acid treatment. The removal of the precipitate may be carried out using a filter press, a drum filter, or the like. However, the present disclosure is not limited thereto, and the removal of the precipitate may be carried out in a general manner capable of removing the precipitate. Through the acid treatment process, the pH of the broth may be reduced to about 1 to 3. For example, the pH thereof may be reduced to a value from about 1.5 to about 2.5. The pH thereof may be reduced to a value from about 1.5 to 2.0.

### Process 7: Reducing Ionic Components in Fermentation Broth (Electrodialysis and Ion Exchange)

The method for obtaining the compound of the present disclosure may include a step of reducing an ionic component in a fermentation broth. In this case, the fermentation broth of Process 7 may be a treated fermentation broth that has been subjected to Process 2 and/or Process 3. Alternatively, the fermentation broth of Process 7 may be a treated fermentation broth that has been subjected to Process 2 and/or Process 3, and then subjected to one or more of Processes 4 to 6, or 8. In one embodiment, a small amount of ions remaining after the process 6 may be further removed in the process 7. In the process 7, an ionic component remaining in the fermentation broth may be separated therefrom by applying a process such as electrodialysis, ion exchange, and SMB chromatography. The ions may be sulfate ions, phosphate ions, sodium ions, ammonium ions, calcium ions, potassium ions, magnesium ions, and the like. Thus, the content of the ionic components may be reduced to, for example, a range of about 20,000 ppm or smaller, such as about 19,000 ppm or smaller, such as about 18,500 ppm or smaller, such as about 18,000 ppm or smaller, such as about 17,500 ppm or smaller, such as about 17,000 ppm or smaller, such as about 16,000 ppm or smaller, such as from about 100 to 15,500 ppm, such as from about 150 to 15,000 ppm. In one embodiment, the reduced concentration of the ionic component may be greater than 0 ppm, such as greater than 10 ppm, such as greater than 20 ppm, such as greater than 50 ppm.

### Process 8: Increasing Concentration of 3-HP in the Fermentation Broth

The method for obtaining the compound of the present disclosure may include a step of increasing the concentration of 3-HP in the fermentation broth having the reduced content of the ionic component. In this case, the fermentation broth of Process 8 may be a treated fermentation broth that has been subjected to Process 2 and/or Process 3. Alternatively, the fermentation broth of Process 8 may be a treated fermentation broth that has been subjected to Process 2 and/or Process 3 and then subjected to one or more of Processes 4 to 7. In this case, the concentration of 3-HP in the treated fermentation broth having the reduced content of the ionic component may be increased by using a method such as evaporation and concentration.

In an embodiment, the concentration of 3-HP may be increased by evaporating and/or concentrating the fermentation broth free of or having a reduced concentration of other ingredients other than 3-HP. In one embodiment, the concentration of 3-HP may be increased by performing the process 8 after performing the processes 1 to 7, and in this case, the concentrated fermentation broth may be an aqueous solution in which almost all impurities except for water have been removed, that is, a 3-HP solution. In this case, the concentration of 3-HP in the concentrated fermentation broth in the form of the aqueous solution may be, for example, in a range of 15 wt % or greater, such as 16 wt % or greater, such as 17 wt % or greater, such as 18 wt % or greater, such as 19 wt % or greater, such as 20 wt % or greater. In this regard, the concentration of 3-HP in the concentrated fermentation broth in the form of the aqueous solution may be about 80 wt % or smaller. In this case, evaporation and concentration may be performed by applying vacuum and/or heat thereto. The concentration of 3-HP may be adjusted by evaporating and removing water.

For example, when performing the step of distilling the fermentation broth using vacuum distillation to increase the concentration of 3-HP, this distillation may be performed for example, in a range of about 10 to 300 mbar, such as about 25 to 280 mbar, such as about 30 to 250 mbar, such as about 50 to 200 mbar, such as about 70 to 190 mbar, such as about 80 to 180 mbar based on the absolute pressure. In some embodiments, the step of increasing the concentration of 3-HP using the vacuum distillation may be performed, for example, at a pressure in a range of about 20 mbar or lower, such as about 25 mbar or lower, such as about 30 mbar or lower, such as about 50 mbar or lower, such as about 70 mbar or lower, such as about 80 mbar or lower. In one embodiment, the step of increasing the concentration of 3-HP using the vacuum distillation may be performed, for example, at a pressure in a range of about 300 mbar or lower, such as about 280 mbar or lower, such as about 250 mbar or lower, such as about 200 mbar or lower, such as about 190 mbar or lower, such as about 180 mbar or lower.

The distillation may be performed by using an oil under membrane distillation apparatus, a wiper type thin film evaporation apparatus, a thin film evaporation apparatus, a centrifugal molecular distillation apparatus, a thin film rising type evaporation apparatus, and the like. However, the present disclosure is not limited thereto, and the distillation may be performed using a multiple effect evaporator. A thermal recompression and mechanical recompression process may be applied to the distillation for energy efficiency.

### Process 9: Collection

The method for obtaining the compound of the present disclosure may further include a step of collecting 3-HP from the fermentation broth. In this case, the fermentation broth of Process 9 may be a treated fermentation broth that has been subjected to Process 2 and/or Process 3 and then at least one of Processes 4 to 8. 3-HP may be collected in an aqueous solution state.

### Method of Obtaining Compound of Present Disclosure

The method for obtaining the compound of the present disclosure may perform all of the above-described steps, may perform selected two or more steps, or may perform selected three or more steps. According to the present disclosure, the steps as described herein may be performed in an order different from the order defined herein. For example, after the filtration, the concentration of pH in the fermentation broth may be increased. Alternatively, the concentration of pH in the fermentation broth may be increased, and then filtration may be performed. In addition, the method of the present disclosure may further include further steps other than the steps described herein.

### Examples

The advantages and features of the present disclosure, and methods of achieving them, will be apparent with reference to examples described below in detail. However, the present disclosure is not limited to the examples disclosed below but may be implemented in various forms different from each other. The following examples are provided to completely inform the scope of the disclosure to a person skilled in the art to which the present disclosure pertains, and the present disclosure is defined by the scope of the claims.

### <Experimental Example 1> Process 2: Separation Test of Insoluble Material

A 3-HP fermentation broth was prepared through batch fermentation in a medium containing glycerol as only carbon source using recombinant E coli. As a result of analyzing supernatant of the fermentation broth using a liquid chromatography method, 3-HP 100 g/L, 1,3-PDO 0.5 g/L, and 0.3 g/L of acetic acid were contained therein, and glycerol was present therein in an amount of 0.1 g/L or smaller.

The fermentation broth was filtered with a porous polymer membrane to separate the insoluble substance therefrom. As a result, 91% of 3-HP (3-HP amount in the filtered fermentation broth relative to 3-HP amount in the fermentation broth before filtering) relative to the initial fermentation broth put into the membrane was collected.

Thereafter, about 10% DI-water (deionized water) based on the initial fermentation broth amount (based on volume) was added to the remaining fermentation broth subjected to the filtration, and then, the broth was stirred, and filtered again. This is because a dead volume for operation (a space required for apparatus operation, and thus, the fermentation broth in the space is not filtered) is present in the filtration apparatus, and thus additional water injection and subsequent filtration processes are additionally required.

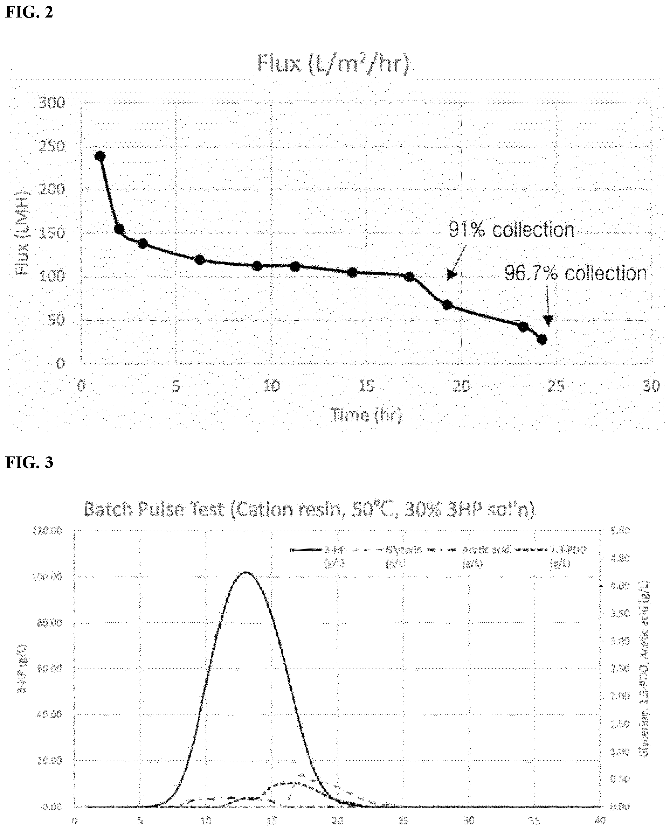

As a result, the collection percentage of 3-HP was increased up to 96.7% with respect to the amount of 3-HP contained in the initial fermentation broth. That is, the collection percentage of 3-HP may be increased up to 96.7% by first obtaining a filtrate having 91% of 3-HP with respect to the amount of 3-HP contained in the initial fermentation broth and then adding a certain amount of water thereto, and then, additionally obtaining a filtrate of having 96.7% of 3-HP with respect to the amount of 3-HP contained in the initial fermentation broth (FIG. 2).

### <Experimental Example 2> Process 5: Identification of 1,3-propanediol and glycerol peaks via single column chromatography

The filtered fermentation broth prepared in Experimental Example 1 was concentrated to prepare about 30 w/w % 3-HP solution, which in turn was used as the feed of Experimental Example 2. 3-HP was contained in a form of calcium salt in the feed. A 380 ml glass column was fully filled with cation exchange resin and, and then, 20 ml of a 30% 3-HP fermentation broth was input thereto at a pulse using a metering pump. While DI-water was used as an eluent, the 3-HP fermentation broth passed through the cation exchange resin column from a bottom upwardly at 10 ml/min. The 3-HP fermentation broth having passed through the column was collected into 10 mL of a sample which was analyzed via liquid chromatography (being treated at a space velocity of 1 hr⁻¹ at 50°C with single column chromatography).

As a result, it was identified that peaks of 1,3-PDO and glycerol were similarly formed near a tail of 3-HP (FIG. 3). Therefore, it may be inferred that the method of the present disclosure may separate 3-HP from 1,3-PDO and glycerol through the SMB chromatography using a repulsive force between 3-HP and the resin.

Further, an SMB chromatography experiment was performed while applying the same ion exchange resin and a 3-inch diameter column. A feed of 300 g/L of 3-HP prepared by ultrafiltration and concentration of the fermentation broth containing the 3-HP was injected into the SMB device. While the eluent (water) was being injected thereto at a content of 1 to 3 times (by weight) of the weight of the feed while maintaining the temperature of the feed at 25 to 50°C, the ratio (= E/R ratio) of Extract and Raffinate flows was adjusted to a value between 0.1 to 5.0, such that glycerol and 1.3-propanediol were removed from the Raffinate flow such that the contents thereof were 30 ppm or smaller. In this regard, the concentration of 3-HP in Raffinate was in a range of 70 g/L to 250 g/L. In addition, when the residual amount of 1.3-propanediol was increased to 50 ppm, a collection percentage of 99.4% (3-HP amount output in the Raffinate flow with respect to the amount of 3-HP injected as a feed) could be achieved.

### <Experimental Example 3> Process 6: Calcium ion removal test

A 20 w/v % 3-HP solution was prepared through a concentration process of the filtered fermentation broth prepared in Experimental Example 1, and was used as the feed of Experimental Example 3. As a result of the ion chromatography, the concentration of Ca ions was about 40,000 ppm, and the concentration of 3-HP in the feed was 213 g/L.

1300 g of the feed (3-HP fermentation broth) was added to a glass beaker, and then about 870g of a 15% sulfuric acid solution was added thereto at a rate of 30 mL/min using a metering pump, followed by stirring at 200 rpm. The sulfuric acid solution was entirely added thereto, followed by further stirring for about 1 hour. The resulting white calcium sulfate particles were vacuum-filtered with a 0.2 micro filter.

As a result, a filtrate of pH 2 to 3 in which 130 g/L of 3-HP was contained, and from which a significant amount of Ca residue was removed was obtained. About 290g of a white solid in a wet state remained on an upper end of a filter paper. The white solid was gypsum containing about 40 wt % or greater of moisture.

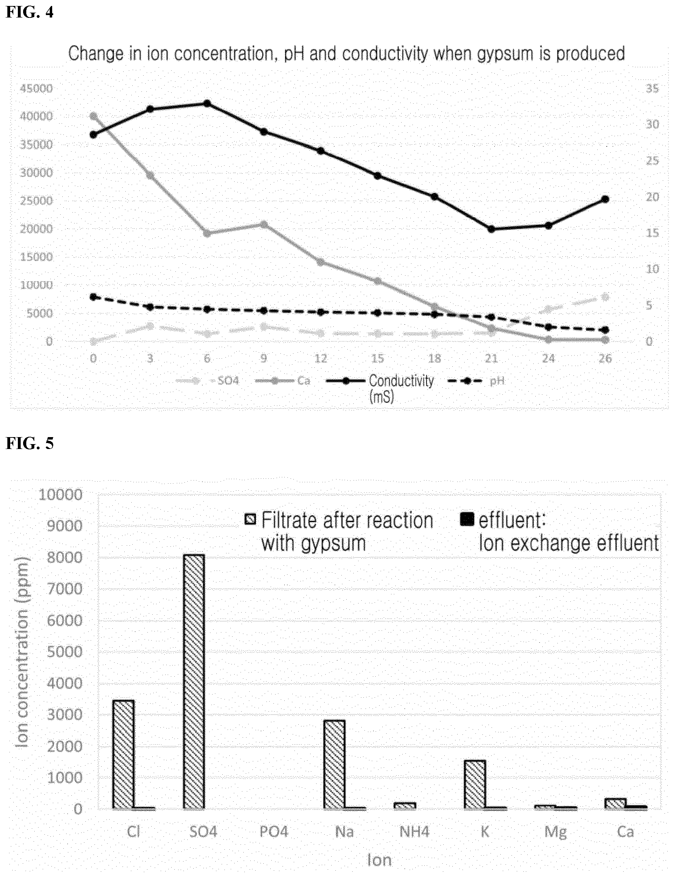

The progress of the precipitation reaction could be identified based on a result of monitoring pH and electrical conductivity. It was identified that after the precipitation process had been completed, the concentration of Ca ions in the filtrate was 500 ppm or smaller (FIG. 4).

### <Experimental Example 4> Process 7: ion exchange test

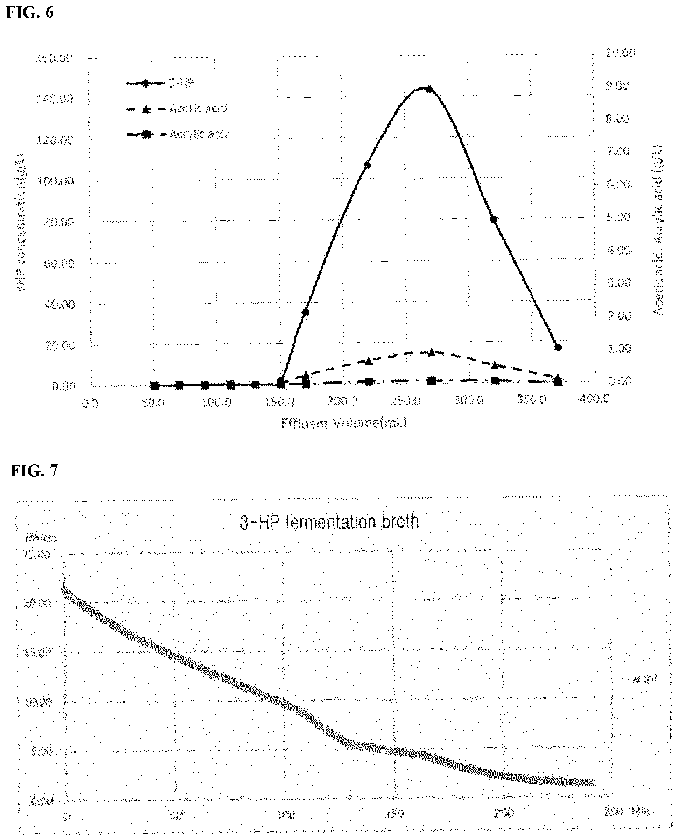

3-HP in the fermentation broth having the pH lowered through Experimental Example 3 may be present in the form of a non-dissociation acid, and ions remaining in the fermentation broth may be additionally removed through ion exchange resin treatment. Calcium was removed from the filtered fermentation broth prepared in Experimental Example 1 via a sulfuric acid reaction as in Experimental Example 3 and then, the calcium-free fermentation broth was converted to a feed having an ion content of about 16,600 ppm, which in turn was used as the feed of Experimental Example 4. As a result, when the feed 250 M was injected into each of the cation exchange resin 65 mL and the anion exchange resin 40 mL at a flow rate of 5 mL/min, 3-HP and the organic acid in the form of the non-dissociation form passed through the ion exchange resin, and the final ion concentration was identified to be about 200 ppm (FIG. 5). Therefore, although the amount of 3-HP was decreased from initial 125 g/L to 75 g/L due to the amount of the water inputted for collecting 3-HP in the ion exchange resin passing-through process, 98% removal of ions in the feed was achieved FIG. 6).

There were ions that have not been removed via one-step ion exchange in which the fermentation broth passed through the cation exchange resin and the anion exchange resin once. These ions were divalent cations (M, Ca) and potassium, which may be removed via additional treatment with a small amount of cation exchange resin.

### <Experimental Example 5> Process 7: Electrodialysis Test

The filtered fermentation broth prepared in Experimental Example 1 was subjected to the treatment process of Experimental Example 3 and then was used as the feed of Experimental Example 5. The ion content in the feed was about 16,600 ppm. Since the concentration of ions to be removed is 3000 ppm or higher, it is preferable to remove major ionic components by applying electrodialysis or SMB chromatography to efficiently remove ionic components from the fermentation broth. The ionic component in the fermentation broth may be removed therefrom via any one of electrodialysis, ion exchange, and SMB chromatography. However, it is preferable to increase the efficiency of ion removal via a combination of two or more processes.

The ionic components remaining in the fermentation broth as the feed 1L having an ion content of about 16,600 ppm were removed using an electrodialysis device equipped with a heterogeneous ion exchange membrane (10 sets: 10 cation exchange membranes and 10 anion exchange membrane being stacked on top of each other in an alternate manner). The fermentation broth flowed through the cation exchange membranes and the anion exchange membranes and the flow rate thereof was 0.6 GPM (2.27 LPM). A voltage of 8V was applied across the ion exchange membranes. The change in the amount of ions in the fermentation broth may be known based on the change in the electrical conductivity of the fermentation broth. As a result of performing the electrodialysis for 4 hours, the electrical conductivity of the fermentation broth was decreased from 21.37 mS/cm to 1.35 mS/cm. This indicated that removal of ions by about 95% was achieved (FIG. 7)

### Industrial Applicability

The present disclosure relates to a method for obtaining a target compound from a fermentation broth, the method comprising:
removing at least a portion of an insoluble substance or a macromolecule from the fermentation broth;
performing at least one of following steps (a) to (c): (a) increasing a concentration of the target compound in the fermentation broth; (b) reducing a concentration of a residual carbon source or alcohol in the fermentation broth; and (c) reducing a concentration of an ionic component in the fermentation broth; and
collecting the target compound from the fermentation broth.

## Claims

1. A method for obtaining a target compound from a fermentation broth, the method comprising:
removing at least a portion of an insoluble substance or a macromolecule from the fermentation broth;
performing at least one of following steps (a) to (c):
(a) increasing a concentration of the target compound in the fermentation broth;
(b) reducing a concentration of a residual carbon source or alcohol in the fermentation broth; and
(c) reducing a concentration of an ionic component in the fermentation broth; and
collecting the target compound from the fermentation broth.

2. The method of claim 1, wherein at least one of the (a) to (c) is repeated twice or more.

3. The method of claim 1, wherein the (a) is performed using at least one selected from a group consisting of evaporation, concentration, and distillation.

4. The method of claim 1, wherein the (c) is carried out via following (c)' or (c)":
(c)': a process of acidifying the fermentation broth to precipitate the ionic component and then removing the precipitated ionic component; and
(c)": a process of treating the fermentation broth using at least one selected from a group consisting of electrodialysis, ion exchange, and chromatography.

5. A method of claim 4, wherein the chromatography of the (c)" is SMB (Simulated Moving Bed) chromatography.

6. The method of claim 1, wherein the insoluble substance includes cells.

7. The method of claim 1, wherein the macromolecule is a protein, a polysaccharide or a lipid.

8. The method of claim 1, wherein the target compound is an organic acid produced by microbial fermentation.

9. The method of claim 1, wherein the carbon source of the (b) is glycerol or glucose.

10. The method of claim 1, wherein the alcohol of the (b) is an alcohol produced by microbial fermentation.

11. The method of claim 1, wherein the alcohol of the (b) is 1,3-propanediol.

12. The method of claim 1, wherein the (b) is carried out by applying the fermentation broth to SMB (simulated moving bed) chromatography.

13. The method according to claim 1, wherein the method includes
removing at least a portion of the insoluble substance or the macromolecule from the fermentation broth;
(a) first increasing the concentration of the target compound in the fermentation broth;
(b) reducing the concentration of the remaining carbon source or alcohol in the fermentation broth;
(c)' acidifying the fermentation broth to precipitate the ionic component and then removing the pre precipitated ionic component;
(c)" treating the fermentation broth using at least one selected from a group consisting of electrodialysis, ion exchange, and chromatography;
(a) second increasing the concentration of the target compound in the fermentation broth; and
collecting the target compound from the fermentation broth.
